Europäisches Patentamt

European Patent Office

Office européen des brevets

(19)

(11) Numéro de publication: **0 216 691**
**B1**

(12) # FASCICULE DE BREVET EUROPEEN

(45) Date de publication du fascicule du brevet:
**31.01.90**

(51) Int. Cl.⁴: **A61M 1/14**

(21) Numéro de dépôt: **86401974.0**

(22) Date de dépôt: **09.09.86**

(54) **Dispositif de raccordement pour circuit de circulation extra-corporelle.**

(30) Priorité: **16.09.85 FR 8513703**

(43) Date de publication de la demande:
**01.04.87 Bulletin 87/14**

(45) Mention de la délivrance du brevet:
**31.01.90 Bulletin 90/5**

(84) Etats contractants désignés:
**AT BE CH DE GB IT LI LU NL SE**

(56) Documents cités:
**EP-A- 0 130 315**
**DE-A- 1 566 619**
**US-A- 4 216 860**

(73) Titulaire: **Charvin, Guy, Parc Saint Honoré Chemin de la Peyrigoue, F-06600 Antibes(FR)**

(72) Inventeur: **Charvin, Guy, Parc Saint Honoré Chemin de la Peyrigoue, F-06600 Antibes(FR)**

(74) Mandataire: **Polus, Camille et al, c/o Cabinet Lavoix 2, Place d'Estienne d'Orves, F-75441 Paris Cedex 09(FR)**

ACTORUM AG

## Description

La présente invention concerne les circuits de circulation extra-corporelle (C.E.C.) assurant la liaison, par l'intermédiaire d'un dispositif de raccordement formé essentiellement de tubes et leurs accessoires -dits lignes en terme de métier- entre un malade et un système coeur-poumon artificiel lors d'une intervention chirurgicale nécessitant l'oxygénation extra-corporelle du sang.

Ce dispositif comporte les lignes essentielles dites ligne artérielle et ligne veineuse, et des lignes auxiliaires telles que lignes d'aspiration, ligne de cardioplégie, ligne de décharge gauche, qui sont traditionnellement présentées, séparément, ou regroupées en tout ou partie, dans un emballage stérile. Juste avant la mise en circulation extra-corporelle du malade, cet emballage de protection de la stérilité est ouvert et le chirurgien ou son aide opératoire remet au technicien de circulation extra corporelle les extrémités des lignes qui doivent être branchées sur le système d'oxygénation coeur-poumon artificiel. On procède notamment ainsi au remplissage de la ligne artérielle et de la ligne veineuse, toutes les deux reliées en circuit clos pour former ce qu'on appelle communément le shunt artério-veineux, au moyen d'un liquide tel que soluté, sang d'appoint, etc... Il faut ensuite assurer l'élimination totale des bulles d'air formées pendant la phase de remplissage de ce shunt. Ce n'est qu'après cette phase de débullage que le chirurgien peut, après avoir scindé le shunt artério-veineux, brancher au moins une canule artérielle sur l'extrémité libre de la ligne artérielle et une/ou des canule(s) veineuse(s) sur l'extrémité libre de la ligne veineuse. Il convient alors d'opérer le remplissage et le débullage des canules.

Le remplissage de toute canule intravasculaire s'effectue, traditionnellement, à partir d'une canule nue, en introduisant son extrémité distale dans le vaisseau concerné du malade : le sang, sous l'effet de sa pression, remplit la canule jusqu'à son embase proximale.

Le débullage de ladite canule s'effectue, ensuite, au niveau de son embase proximale, par divers dispositifs ou moyens qui éliminent tout air résiduel de la canule et empêchent l'ajout d'air extérieur au moment précis du branchement de la canule sur sa ligne extra-corporelle.

De même, il existe des dispositifs qui, après cette opération de branchement, permettent d'éliminer tout air résiduel malencontreusement piégé dans le circuit fermé.

Sur le plan pratique, ces opérations classiques de remplissage de canules, de branchement et de débullage, sont longues et délicates et nécessitent l'utilisation de différents acccessoires. En effet, lors de la scission par le chirurgien du shunt artério-veineux pour libérer les extrémités distales de la ligne artérielle et de la ligne veineuse, chacune de ces lignes doit être au préalable clampée pour éviter tout écoulement du liquide de remplissage. De même, le chirurgien doit, après introduction de la canule intravasculaire dans le vaisseau et son remplissage, clamper son extrémité proximale pour empêcher l'échappement du sang à l'extérieur. Il doit, enfin, brancher chaque canule sur l'extrémité distale de la ligne correspondante en faisant attention de ne pas piéger de l'air à l'intérieur du circuit. L'un des moyens traditionnellement utilisé pour empêcher l'apport d'air dans le circuit, lors du branchement de la canule sur sa ligne, consiste à faire couler un liquide (tel que soluté stérile) conjointement sur l'orifice de l'embase proximale de la canule et sur l'orifice distal de la ligne au moment précis du branchement de la canule sur la ligne, de manière qu'il y ait un trop plein simultané de liquide au niveau de ces deux orifices et empêcher le piégeage d'air lors de la connexion. L'un des moyens utilisés traditionnellement pour éliminer les bulles d'air malencontreusement piégées après l'opération de connexion canule/ligne, consiste à utiliser un connecteur de liaison canule-ligne qui possède latéralement une ouverture sous la forme d'une embase à cône luer femelle sur laquelle le chirurgien pourra brancher une seringue permettant d'aspirer les bulles d'air résiduel. Une fois cette opération terminée, le chirurgien obturera cette embase femelle à l'aide d'un bouchon verrouillable à cône luer mâle.

Il est à noter que l'opération de débullage de la canule artérielle est beaucoup plus lourde de conséquences que celle de débullage de la canule veineuse. En effet, pour cette dernière, la présence de bulles d'air résiduel n'a pas de conséquences majeures puisque le sang veineux sort du malade pour aller dans l'oxygénateur artificiel et ses dispositifs aval de piégeage de bulles d'air. Par contre, le débullage et le branchement de l'embase proximale de la canule artérielle sur l'extrémité distale de la ligne artérielle sont des opérations difficiles et à risques : il est impératif qu'il n'y ait aucune bulle d'air dans cette partie du circuit puisque le liquide de remplissage contenu initialement dans la ligne artérielle et le sang artériel contenu initialement dans la canule artérielle seront propulsés, tous les deux, sous l'action de la pompe de circulation extra-corporelle, directement dans l'artère du malade (généralement l'aorte).

Par ailleurs, il est extrêmement important que les canules artérielle(s) et veineuse(s) soient parfaitement solidaires de leurs lignes : notamment, l'embase proximale de la canule artérielle doit être particulièrement bien fixée sur l'extrémité distale de la ligne artérielle du fait de la pression générée par la pompe. Le chirurgien utilise, en général, pour assurer une bonne fixation, des liens spéciaux, serrés à l'aide d'une pince appropriée, permettant le blocage des canules sur les connecteurs distaux des lignes. Cette opération demande, bien sûr, un temps supplémentaire et est quelquefois délicate.

En résumé, les équipements de circuits distaux traditionnels : canules isolées, shunt artérioveineux simple, connecteurs et seringues de débullage, clamps multiples, liens, pinces, etc... nécessitent des manipulations qui rallongent sensiblement le temps opératoire, et qui, de surcroit, comporte des risques non négligeables d'introduction d'air dans le système vasculaire (consécutifs à un mauvais débullage) ou des risques de débranchement ultérieur, en cours de circulation extra-corporelle, d'une canule (consécutif à un branchement extemporané hâtif de celle-ci sur l'extrémité distale de sa ligne).

L'invention se propose de remédier à tous ces inconvénients. Elle a pour objet à cet effet un dispositif de raccordement pour circuit de circulation extracorporelle comprenant une ligne artérielle et une ligne veineuse, dont la liaison entre elles et en circuit fermé sur le système coeur-poumon artificiel forme le shunt artério-veineux, caractérisé en ce que la liaison des extrémités distales de la ligne artérielle et de la ligne veineuse se fait par au moins une canule artérielle reliée de façon amovible à son extrémité distale à la ligne veineuse.

Cette liaison amovible peut être assurée par un manchon creux élastique en forme de U ou de V enfilé à une extrémité sur la canule artérielle et à son autre extrémité, directement ou par l'intermédiaire d'un connecteur, sur la ligne veineuse ou encore sur l'extrémité distale d'une canule veineuse fixée par son extrémité proximale sur la ligne veineuse.

Il est en effet possible de monter selon l'invention dans le shunt artério-veineux, non seulement la ou les canule(s) artérielle(s), mais encore la ou les canule(s) veineuse(s). Lorsque deux canules veineuses sont ainsi prévues, l'organe de liaison, au lieu d'être un simple manchon, est à trois voies, deux des voies étant destinées à être enfilées sur les canules veineuses. En variante, les deux canules veineuses ne sont pas incorporées dans le shunt artério-veineux, mais celui-ci comporte un connecteur à trois voies, l'une enfilée dans l'extrémité distale de la ligne veineuse et les deux autres enfilées dans les deux voies restantes de l'organe de liaison et prêtes à recevoir deux canules veineuses après enlèvement de cet organe.

Lorsqu'il y a deux canules artérielles et une canule veineuse, l'organe de liaison est un manchon à trois voies. Lorsqu'il y a enfin deux canulés artérielles et deux canules veineuses, l'organe de liaison est un manchon à quatre voies, ou, selon une autre réalisation, il y a deux manchons à une voie reliant chacun l'extrémité distale d'une canule artérielle à l'extrémité proximale d'une canule veineuse ou de son connecteur.

Ce dispositif de raccordement perfectionné permet le remplissage simultané des lignes artérielle et veineuse et de la ou des canule(s) artérielle(s) (et éventuellement de la ou des canule(s) veineuse(s)), avec un liquide de remplissage extérieur, et non plus avec le sang du malade comme c'est le cas avec le matériel traditionnel. De même, le débullage de la ou des canule(s) est réalisé simultanément avec le débullage des lignes artérielle et veineuse, puisque l'ensemble ligne artérielle + canule(s) artérielle(s) + éventuellement canule(s) veineuse(s) + ligne veineuse constitue un circuit fermé, qu'il est très facile de débuller à l'aide du système coeur-poumon artificiel.

Par ailleurs, l'embase proximale de chaque canule peut être fixée originellement sur la ligne d'une manière particulièrement résistante grâce à des moyens industriels (collage, sertissage, liens perfectionnés, écrous spéciaux, etc...). Enfin, selon un mode de réalisation préféré de l'invention, ce dispositif de raccordement perfectionné peut être conditionné dans un système d'emballage gigogne permettant de préserver "stérile" la partie du circuit à chaque stade de son utilisation. Notamment, ce système de conditionnement comporte un emballage permettant de préserver "stérile" la partie du circuit destinée au champ opératoire (portion distale des lignes artérielle et veineuse et la totalité de la ou des canule(s) artérielle(s) et éventuellement de la ou des canule(s) veineuse(s)), tandis que l'autre partie (les tronçons proximaux de ces lignes) peut être branchée par le perfusionniste sur le système coeur-poumon artificiel. Cette dernière réalisation préférentielle de l'invention permet un gain de temps, appréciable pour le chirurgien, notamment dans le cas d'une urgence, où le malade doit être opéré dans les plus brefs délais. En effet, grâce à ce système d'emballage gigogne, le perfusionniste peut, à son niveau, procéder à la mise en marche de l'équipement de circulation extra-corporelle, notamment réaliser le remplissage et le débullage du shunt artério-veineux. Parallèlement à ce travail du perfusionniste, le chirurgien peut commencer l'intervention chirurgicale pour introduire, juste le moment venu, la ou les canule(s) artérielle(s) dans la ou les artère(s) et, le cas échéant, la ou les canule(s) veineuse(s) dans le(s) vaisseau(x) concerné(s). Le chirurgien n'a donc plus à attendre le remplissage et le débullage de la ligne artérielle et de la ligne veineuse ; il n'a plus à remplir la ou le(s) canule(s) intravasculaire(s), il n'a plus à les débuller, il n'a plus à les brancher sur les lignes correspondantes, il n'a plus à les fixer avec des liens spéciaux ou autres moyens de fixation, il n'a plus, enfin, à réaliser l'ultime opération de débullage du tronçon embase de la canule/ extrémité distale de la ligne. Par ailleurs, les opérations de clampage et de déclampage sont divisées par deux. En résumé, le dispositif selon l'invention simplifie à l'extrême les manipulations du chirurgien : celui-ci n'a qu'à introduire la ou le(s) canule(s) dans le ou le(s) vaisseau(x) du malade, après avoir ôté l'organe de liaison distal.

D'autres caractéristiques et avantages de l'invention ressortiront de la description détaillée qui va suivre de quelques-uns de ses modes de réalisation. On se référera pour cela au dessin annexé sur lequel :

La Fig. 1 est une vue en plan de l'un des modes de réalisation du dispositif de raccordement selon l'invention ;

La Fig. 2 est une vue en coupe, à plus grande échelle, de la partie distale d'un dispositif tel que celui de la Fig. 1 ;

La Fig. 3 est une vue analogue à la Fig. 2 d'un autre mode de réalisation ;

La Fig. 4 est une vue analogue à la Fig. 2 d'une autre variante ;

La Fig. 5 représente un dispositif selon l'invention, dans son emballage ;

La Fig. 6 représente ce même dispositif, dans une variante d'emballage, et,

La Fig. 7 est une vue en coupe selon la ligne 7-7 de la Fig. 6.

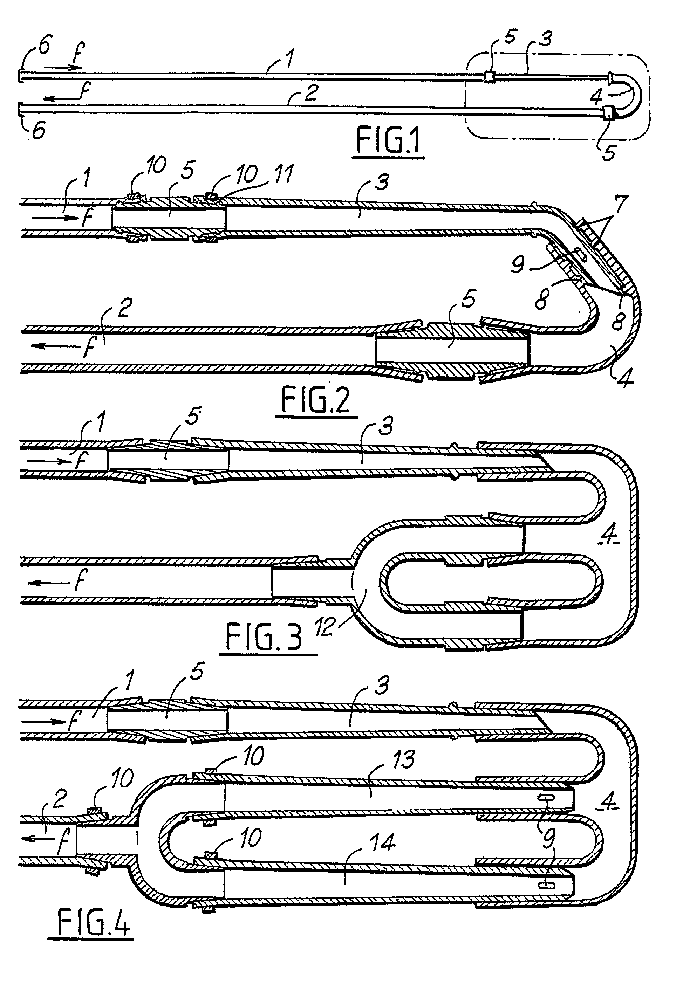

Le dispositif de raccordement représenté sur la Fig. 1 comporte une ligne artérielle 1, dont l'extrémité distale est reliée à une canule artérielle 3, elle-même reliée à son extrémité distale à un organe de liaison 4 formé par un manchon creux en forme de U, relié à une ligne veineuse 2. Les liaisons ligne artérielle 1/canule artérielle 3 et ligne veineuse 2/organe de liaison 4, sont assurées par des connecteurs simples à une voie 5. Les extrémités proximales des lignes sont protégées préférentiellement par des bouchons 6 que le perfusionniste devra ôter pour brancher le dispositif sur le sytème coeur-poumon artificiel. Une fois ce branchement effectué, ce qui boucle le shunt artério-veineux, celui-ci est rempli avec un liquide de remplissage (soluté, sang extérieur, etc...) qui sera propulsé selon le sens des flèches f : départ par la ligne artérielle 1, retour par la ligne veineuse 2. Après le temps requis par l'opération de recirculation du liquide de remplissage, nécessaire au débullage complet du shunt, le chirurgien, après avoir clampé les lignes 1 et 2, devra ôter l'organe de liaison 4, puis simplement introduire la canule artérielle 3 dans l'artère du malade, il devra, par ailleurs, brancher la ou le(s) canule(s) veineuse(s) sur l'extrémité distale de la ligne veineuse. Après le déclampage des lignes 1 et 2, le malade sera mis en circulation extra-corporelle.

La Fig. 2 représente, en coupe, la partie distale du shunt entouré d'un trait mixte sur la Fig. 1. Dans cette Figure, la canule artérielle 3 comporte une petite variante : son extrémité distale est inclinée par rapport à son axe principal. Par ailleurs, elle comporte un ou plusieurs trous latéraux 9. L'organe de liaison 4 est en forme de V et non de U pour tenir compte de la forme de l'extrémité distale de la canule. Cette figure met en évidence le détail des connecteurs 5 qui comportent, preférentiellement, des segments d'étanchéité 11 permettant une très bonne fixation de la ligne 1, de la canule 3, du manchon 4 et de la ligne 2. Les branchements de la ligne 1 et de la canule 3 sont renforcés par la présence de deux liens de fixation 10.

En ce qui concerne l'organe de liaison 4, celui-ci comporte des segments d'étanchéité circulaires 7, ainsi qu'un autre segment d'étanchéité 8, de forme approximativement elliptique, évitant tout risque d'espace mort à l'extrémité distale de la canule 3, dans lequel pourraient stagner des bulles d'air.

Selon un autre mode de réalisation de l'invention, non représenté, mais aisément déductible du mode de réalisation des Fig. 1 ou 2, la ligne veineuse 2 peut être directement branchée sur une canule veineuse par, éventuellement, l'intermédiaire du connecteur 5 : dans ce cas, l'embase proximale de la canule veineuse est branchée sur le connecteur 5 de la ligne veineuse, alors que son extrémité distale est branchée sur l'organe de liaison 4.

La Fig. 3 représente une variante, dans laquelle le connecteur à une voie 5 de la ligne veineuse 2, a été remplacé par un connecteur à trois voies 12, relié à un organe de liaison 4 lui-même à trois voies. Avec un tel agencement, le chirurgien peut brancher directement deux canules veineuses sur les deux branches parallèles ou en Y du connecteur 12.

La Fig. 4 représente une variante du mode de réalisation de la Fig. 3 dans laquelle sont montées deux canules veineuses 13 et 14. Ces deux canules peuvent comporter, accessoirement, des trous latéraux 9. Par ailleurs, elles peuvent être solidarisées sur le connecteur 12 par collage, par des écrous, par des liens de serrage 10 ou par tout autre moyen.

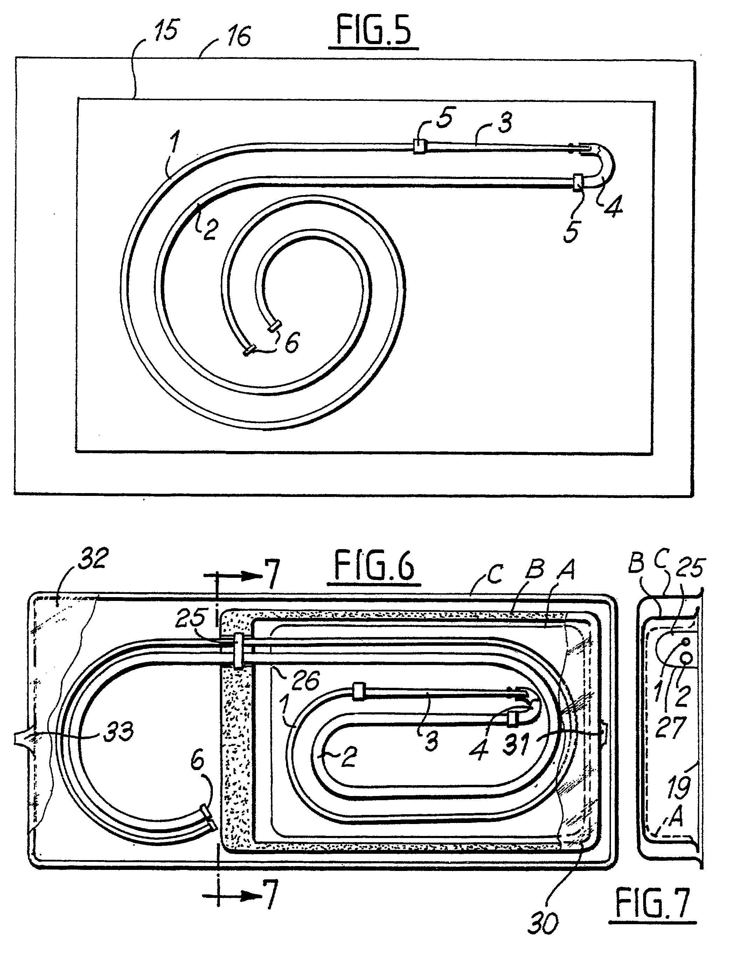

La Fig. 5 représente un dispositif de raccordement selon l'invention, qui est conditionné dans au moins deux emballages assurant la stérilité de l'ensemble, jusqu'à l'utilisation finale du dispositif par le chirurgien.

Dans ce système. l'emballage extérieur 16 enlevé, l'emballage intérieur 15 préservé stérile peut être introduit en bloc opératoire, voire même disposé tel que sur le champ opératoire. Ce n'est qu'après l'ouverture de l'emballage 15 que le chirurgien pourra remettre au perfusionniste les extrémités proximales des lignes 1 et 2, protégées par des bouchons 6, alors qu'il gardera sur le champ opératoire la partie distale du dispositif contenant la canule artérielle 3.

La Fig. 6 représente un dispositif selon l'invention, conditionné dans un système de trois emballages gigognes, selon un mode de réalisation préféré de l'invention.

Ces trois emballages gigognes préservent la stérilité totale ou partielle du shunt artério-veineux à chaque stade de sa mise en oeuvre et sont désignés de l'intérieur vers l'extérieur par A, B et C d'une manière générale.

L'emballage intérieur A, qui est accessible après ouverture de l'emballage intermédiaire B, est avantageusement, comme représenté, une simple barquette ou plateau, sans couvercle sur lequel sont enroulées les extrémités des lignes 1 et 2 destinées au champ opératoire avec la canule 3 et le manchon 4.

L'emballage intermédiaire B, qui est accessible après ouverture de l'emballage extérieur C, est avantageusement, comme représenté, une barquette comportant un joint d'étanchéité 25 (voir Fig. 7) amovible, au travers duquel passent les lignes artérielle 1 et veineuse 2 débouchant de l'emballage A par une échancrure 26. Le joint 25 est lui-même engagé à friction dans un alvéole 27 de la barquette B. Le joint 25 est réalisé dans un matériau élastomère, tel que mousse de polyéthylène ou de polyester ou de polyuréthane, caoutchouc ou silicone, ou tout autre matériau permettant une bonne conformation du joint à son logement 26 et assurant la fixation dans ce dernier par serrage. La barquette B est fermée par un couvercle 30, qui se présente, préférentiellement, sous la forme d'un film pelable transparent fixé par soudage ou collage sur le pourtour de la barquette et détachable au moyen d'une languette 31.

L'emballage extérieur C, logeant la partie des lignes 1 et 2 raccordable au coeur-poumon artificiel, est représenté sous la forme d'une barquette obturée sur sa partie supérieure d'un couvercle ou film pelable 32 avec languette d'ouverture 33. Les extrémités proximales des lignes sont protégées par des capuchons 6. L'emballage C peut cependant être tout autre dispositif de conditonnement permettant la protection totale de la stérilité de son contenu, tel qu'un sac fermé étanche aux bactéries.

La chronologie de préhensions et d'ouvertures de ces trois emballages et la mise en oeuvre de l'équipement selon l'invention sont les suivantes :

1) préhension et ouverture de l'emballage extérieur C par le perfusionniste ;

2) préhension, par le perfusionniste, de la partie des lignes artérielle 1 et veineuse 2 qui sort de l'emballage B, puis branchement des extrémités proximales de ces lignes, après enlèvement des capuchons de protection 6, sur le système coeur-poumon artificiel.

3) remplissage et débullage du shunt artério-veineux. Pendant cette opération, la partie du shunt artérioveineux destinée au champ opératoire dans son emballage A est toujours préservée intégralement stérile, enfermée dans l'emballage B, d'une manière étanche aux contaminations extérieures grâce au joint 25 et au couvercle 30.

Par ailleurs, le couvercle 30, préférentiellement transparent, permet de contrôler qu'il ne reste pas de bulles dans le shunt artério-veineux en fin d'opération de débullage.

4) ouverture de l'emballage intermédiaire B, par l'enlèvement du couvercle 30. Cette ouverture, réalisée par le perfusionniste ou un autre assistant du chirurgien, se fait sans faute d'asepsie, notamment dans le cas d'un couvercle sous forme de film pelable muni d'une languette 31.

5) préhension de l'emballage A par le chirurgien ou son assistant équipé de gants stériles, puis enlèvement de la partie stérile du shunt artério-veineux destiné au champ opératoire, sans faute d'asepsie : même si le shunt artério-veineux touche le bord extérieur de l'emballage A, celui-ci ayant été conservé intégralement stérile, il n'y a pas de contamination possible dudit shunt.

6) après clampage des lignes, rupture du shunt artério-veineux par le chirurgien qui désolidarise la ligne artérielle 1 avec sa canule 3 de la ligne veineuse 2, suivie de l'introduction, par le chirurgien, de la canule artérielle dans l'artère du malade et du branchement de la canule veineuse (ou des deux canules veineuses) sur l'extrémité distale de la ligne veineuse 2, lorsque ces canules, comme c'est le cas pour le dispositif illustré aux Figs. 6 et 7, n'y sont pas déjà incorporées.

Le malade peut alors être mis en circulation extra-corporelle après déclampage des lignes.

Il est à noter que les systèmes d'emballage de stérilité représentés par les figures 5, 6 et 7 sont donnés à titre illustratif et non limitatif, de même que la variante de dispositif indiquée sur ces figures. En effet, ces systèmes d'emballage peuvent contenir d'autres réalisations du dispositif selon l'invention : telles que dispositif avec connecteur trois voies pour double canulation veineuse, ou com¸ prenant en plus de la canule artérielle, une ou deux canule(s) veineuse(s). De même, le dispositif selon l'invention peut inclure un filtre pré-bypass, et le système d'emballage gigogne peut conditionner, en plus du dispositif selon l'invention, des lignes d'aspiration et/ou de cardioplégie et/ou décharge gauche, ou d'autres lignes.

Le dispositif selon l'invention est particulièrement destiné à la mise en circulation extracorporelle de malades subissant des interventions chirurgicales à coeur ouvert, ou qui nécessitent une oxygénation extra-corporelle d'une ou plusieurs parties de leur organisme.

## Revendications

1. Dispositif de raccordement pour circuit de circulation extra-corporelle du sang d'un malade, comprenant une ligne artérielle (1) et une ligne veineuse (2) dont la liaison entre elles et en circuit fermé sur le système coeur-poumon artificiel forme le shunt artério-veineux, caractérisé en ce que la liaison des extrémités distales de la ligne artérielle (1) et de la ligne veineuse (2) se fait par au moins une canule artérielle (3) reliée de façon amovible à son extrémité distale à la ligne veineuse (2).

2. Dispositif selon la revendication 1, caractérisé en ce que la liaison amovible est assurée par un organe formé d'un manchon creux élastique (4) en forme de U ou de V enfilé à une extrémité sur la canule artérielle (3) et, à son autre extrémité, sur la ligne veineuse (2) directement ou par l'intermédiaire d'un connecteur (5) ou d'une canule veineuse (13) montés sur l'extrémité distale de cette ligne.

3. Dispositif selon la revendication 1, caractérisé en ce qu'un connecteur (12) en forme de Y est monté sur l'extrémité distale de la ligne veineuse (2) et la liaison amovible est assurée par un organe à trois voies (4) dont l'une est enfilée sur la canule artérielle (3) et les deux autres sur les deux voies libres du connecteur (12) ou sur les extrémités distales de deux canules veineuses (13, 14) montées respectivement sur ces deux voies du connecteur (12).

4. Dispositif selon la revendication 2 ou 3, caractérisé en ce que l'organe de liaison (4) comporte, au moins sur sa partie artérielle, un ou plusieurs segments d'étanchéité (7, 8).

5. Dispositif selon la revendication 4, caractérisé en ce que ces segments sont circulaires (7) ou d'une configuration approximativement elliptique (8).

6. Dispositif selon l'une quelconque des revendications précédentes, caractérisé en ce que l'extrémité distale de la ligne artérielle (1) est reliée à l'embase proximale de la canule artérielle (3) par un connecteur creux (5).

7. Dispositif selon l'une quelconque des revendications précédentes, caractérisé en ce que la fixation de l'extrémité proximale des canules (3, 13, 14) sur l'extrémité distale des lignes respectives (1 ou 2) ou un connecteur (5, 12) monté sur cette extrémité est assurée par collage, par écrou, et/ou par des liens de serrage (10).

8. Dispositif selon l'une quelconque des revendications précédentes, caractérisé en ce qu'il est conditonné dans au moins deux emballages (15 et 16) protégeant sa stérilité jusqu'à son utilisation finale.

9. Dispositif selon l'une quelconque des revendications 1 à 7, caractérisé en ce qu'il est conditionné dans trois emballages gigognes, qui en assurent la stérilité et dont les caractéristiques sont les suivantes, les emballages étant énumérés de l'intérieur vers l'extérieur : le premier (A) contient la partie du shunt, dont la ou le(s) canule(s) intravasculaire(s) (3, 13, 14), destinée au champ opératoire, le second (B) intermédiaire, enveloppe le premier et forme au point- de-vue de la stérilité écran entre celui-ci et le troisième (C), ce dernier contenant l'emballage (B) et la partie sortante des lignes destinée à être raccordée au système coeur-poumon artificiel.

10. Dispositif selon la revendication 9, caractérisé en ce que l'emballage intermédiaire (B) comporte au moins un point (25) laissant passer la partie des lignes raccordable au système coeur-poumon artificiel, tout en protégeant la stérilité de la partie des lignes destinée au champ opératoire.

## Claims

1. Connecting device for an extracorporeal circulation circuit for a patient's blood, comprising an arterial line (1) and a veinous line (2), the connection between said lines and in closed circuit on the artificial heart-lung system forming the arterio-veinous shunt, characterised in that the distal ends of the arterial line (1) and veinous line (2) are connected by means of at least one arterial cannula (3) which is moveably connected, at its distal end, to the veinous line (2).

2. Device according to claim 1, characterised in that the moveable connection is provided by a member formed from a resilient hollow sleeve (4) in the form of a U or V which is threaded at one end onto the arterial cannula (3) and at its other end onto the veinous line (2) either directly or via a connector (5) or a veinous cannula (13) mounted on the distal end of this line.

3. Device according to claim 1, characterised in that a Y-shaped connector (12) is mounted on the distal end of the veinous line (2) and the moveable connection is provided by a three-way member (4) one way of which is fitted over the arterial cannula (3) whilst the other two are fitted over the two free ends of the connector (12) or the distal ends of two veinous cannulas (13), (14) mounted respectively on these two paths of the connector (12).

4. Device according to claim 2 or 3, characterised in that the connecting member (4) comprises, at least on its arterial portion, one or more sealing segments (7, 8).

5. Device according to claim 4, characterised in that these segments are circular (7) or of substantially elliptical configuration (8).

6. Device according to any one of the preceding claims, characterised in that the distal end of the arterial line (1) is connected to the proximal base of the arterial cannula (3) by a hollow connector (5).

7. Device according to any one of the preceding claims, characterised in that the proximal end of the cannulas (3, 13, 14) is fitted to the distal end of the respective lines (1 or 2) or a connector (5, 12) mounted on this end, by adhesive bonding, by the use of a nut and/or by means of clamping means (10).

8. Device according to any one of the preceding claims, characterised in that it is packed in at least two packages (15 and 16) which keep it sterile until it is finally used.

9. Device according to any one of claims 1 to 7, characterised in that it is packed in three nesting packages, ensuring its sterility and having the following characteristics, the packages being listed from the inside outwards: the first (A) contains the part of the shunt, including the intravascular cannula or cannulas (3, 13, 14), which is intended for the operating area, the second (B) intermediate package surrounds the first and form the point of view of sterility forms a screen between the first and third packages (C), the latter containing the package (B) and the part emerging from the lines which is intended to be connected to the artificial heart-lung system.

10. Device according to claim 9, characterised in that the intermediate package (B) comprises at least one point (25) which lets through the part of the lines which can be connected to the artificial heart-lung system, whilst protecting the sterility of that part of the lines which is intended for the operating area.

## Patentansprüche

1. Anschlußvorrichtung für außerkörperliches Kreislaufsystem des Blutes eines Kranken, mit einer arteriellen Leitung (1) und einer venösen Leitung (2), deren Verbindung untereinander und bei geschlossenem Kreislauf in dem künstlichen Herz-Lungensystem den arterio-venösen Shunt bildet, dadurch gekennzeichnet, daß die Verbindung der distalen Enden der arteriellen Leitung (1) und der venösen Leitung (2) durch wenigstens eine arterielle Kanüle (3) gebildet ist, die entfernbar an deren distalem Ende mit der venösen Leitung (2) verbunden ist.

2. Vorrichtung nach Anspruch 1, dadurch gekennzeichnet, daß die entfernbare Verbindung durch ein Organ sichergestellt ist, das aus einer elastischen hohlen Manschette (4) in Form eines U oder eines V gebildet ist, die an einem Ende auf die arterielle Kanüle (3) und an ihrem anderen Ende auf die venöse Leitung (2) direkt oder mittels eines Verbinders (5) oder einer venösen Kanüle (13) aufgesetzt ist, die an dem distalen Ende dieser Leitung angebracht sind.

3. Vorrichtung nach Anspruch 1, dadurch gekennzeichnet, daß ein Verbinder (12) in Form eines Y an dem distalen Ende der venösen Leitung (2) angebracht ist, und die entfernbare Verbindung durch ein Drei-Wege-Organ (4) sichergestellt ist, von dem einer auf die arterielle Kanüle (3) und die beiden anderen auf den beiden freien Wegen des Verbinders (12) oder auf den distalen Enden der beiden venösen Kanülen (13, 14) aufgesetzt sind, die auf den entsprechenden Wegen des Verbinders (12) angeordnet sind.

4. Vorrichtung nach Anspruch 2 oder 3, dadurch gekennzeichnet, daß das Verbindungsorgan (4) wenigstens auf seinem arteriellen Bereich einen oder mehrere Dichtungsabschnitte (7, 8) aufweist.

5. Vorrichtung nach Anspruch 4, dadurch gekennzeichnet, daß diese Abschnitte kreisförmig (7) oder von einer annähernd elliptischen Ausbildung (8) sind.

6. Vorrichtung nach einem der vorhergehenden Ansprüche, dadurch gekennzeichnet, daß das distale Ende der arteriellen Leitung (1) mit der proximalen Fuß- oder Befestigungsfläche der arteriellen Kanüle (3) durch einen hohlen Verbinder (5) verbunden ist.

7. Vorrichtung nach einem der vorhergehenden Ansprüche, dadurch gekennzeichnet, daß die Befestigung des proximalen Endes der Kanülen (3, 13, 14) auf dem distalen Ende der entsprechenden Leitungen (1 oder 2), oder ein Verbinder (5, 15), der an diesem Ende angebracht ist, durch Kleben, durch Schrauben und/oder durch Zwingen oder Klemmen (10) sichergestellt ist.

8. Vorrichtung nach einem der vorhergehenden Ansprüche, dadurch gekennzeichnet, daß sie wenigstens in zwei Verpackungen (15 und 16) angeordnet ist, die ihre Sterilität bis zu ihrer endgültigen Verwendung schützen.

9. Vorrichtung nach einem der Ansprüche 1 bis 7, dadurch gekennzeichnet, daß sie in drei einschiebbare Verpackungen aufgeteilt ist, die ihr die Sterilität sicherstellen und deren Eigenschaften die folgenden sind, wobei die Verpackungen von innen nach außen numeriert sind: die erste (A) enthält den Bereich des Shunts, deren intravaskuläre Kanüle(n) (3, 13, 14), die für das Operationsfeld bestimmt ist (sind), die zweite Verpackung (B) dazwischen die erste umhüllt und im Hinblick auf die Sterilität zwischen dieser und der Dritten (C) eine Abschirmung bildet, wobei letztere die Verpackung (B) und den austretenden Bereich der Leitungen enthält, der dazu bestimmt ist, mit dem künstlichen Herz-Lungensystem verbunden zu werden.

10. Vorrichtung nach Anspruch 9, dadurch gekennzeichnet, daß die Zwischenverpackung (B) wenigstens einen Punkt (25) aufweist, der den mit dem künstlichen Herz-Lungensystem verbindbaren Bereich der Leitungen hindurchtreten läßt, unter Schützen der Sterilität des Leitungsbereichs, der für das Operationsfeld bestimmt ist.

EP 0 216 691 B1

FIG.1

FIG.2

FIG.3

FIG.4

EP 0 216 691 B1

FIG.5

FIG.6

FIG.7